(19) Europäisches Patentamt
European Patent Office
Office européen des brevets

(11) EP 4 796 217 A1

(12) EUROPEAN PATENT APPLICATION
published in accordance with Art. 153(4) EPC

(43) Date of publication:
**26.08.2026 Bulletin 2026/35**

(21) Application number: **24878847.3**

(22) Date of filing: **30.09.2024**

(51) International Patent Classification (IPC):
***B01J 29/70*** *(2006.01)* ***B01J 29/06*** *(2006.01)*
***C01B 39/04*** *(2006.01)* ***C07D 307/50*** *(2006.01)*

(52) Cooperative Patent Classification (CPC):
**B01J 29/00; B01J 29/06; B01J 29/072; B01J 29/70; B01J 37/04; C01B 39/04; C07D 307/36; C07D 307/50**

(86) International application number:
**PCT/CN2024/122709**

(87) International publication number:
**WO 2025/082203 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311361864**
**19.10.2023 CN 202311359263**

(71) Applicants:
- **China Petroleum & Chemical Corporation**
  **Beijing 100728 (CN)**
- **Sinopec (Shanghai) Research Institute of Petrochemical Technology Co., Ltd.**
  **Shanghai 201208 (CN)**

(72) Inventors:
- **YANG, Weimin**
  **Shanghai 201208 (CN)**
- **HAN, Xiao**
  **Shanghai 201208 (CN)**
- **LI, Xiangcheng**
  **Shanghai 201208 (CN)**
- **WANG, Zhendong**
  **Shanghai 201208 (CN)**
- **FENG, Xinqiang**
  **Shanghai 201208 (CN)**
- **LIU, Chuang**
  **Shanghai 201208 (CN)**

(74) Representative: **Hoffmann Eitle Patent- und Rechtsanwälte PartmbB**
**Arabellastraße 30**
**81925 München (DE)**

# (54) SULFONATION-MODIFIED SCM-36 MOLECULAR SIEVE, AND PREPARATION METHOD THEREFOR AND USE THEREOF

(57) The present disclosure is directed to a modified SCM-36 molecular sieve and a preparation method and use thereof. In the present disclosure, hydrogen-form SCM-36 is used to carry out sulfonate loading, obtaining a modified SCM-36-$SO_3H$ molecular sieve, which has a schematic chemical composition represented by the formula "$mSiO_2 \cdot nAl_2O_3 \cdot pSO_3H$", wherein $22 \leq m/n \leq 80$, and $10 \leq m/p \leq 160$. In the biomass → HMF reaction, the use of modified SCM-36-$SO_3H$ molecular sieve as a solid acid catalyst provides the method with the advantages of simple operation, high product selectivity, good catalyst stability, easy product separation, and easy catalyst recovery, achieving a green and efficient conversion of biomass to 5-hydroxymethylfurfural.

SCM-36-SO3H
Intensity (a.u.)
10
20
30
40
50
60
70
2θ (°)

FIG. 4

## Description

## TECHNICAL FIELD

The present disclosure relates to the field of catalytic chemistry. Specifically, the present disclosure relates to a sulfonation-modified two-dimensional nanosheet SCM-36 molecular sieve and a preparation method and use thereof, particularly the use thereof in catalytic conversion of biomass.

## BACKGROUND ART

In industry, molecular sieve materials are widely used in the fields of catalysis, ion exchange, adsorption, and separation due to their open structure and large surface area. The subtle differences in the structure of these materials indicate differences in various observable properties used to characterize them, such as their morphology, specific surface area, pore size and the variability of these sizes, and also imply significant differences in their catalytic and adsorption properties.

The basic framework structure of crystalline microporous molecular sieves is based on a rigid three-dimensional $TO_4$ ($SiO_4$, $AlO_4$, etc.) unit structure; in this structure, $TO_4$ shares oxygen atoms in a tetrahedral manner, and the charge balance of framework tetrahedra, such as $AlO_4$, is maintained by the presence of surface cations such as $Na^+$ and $H^+$. It can be seen that the framework properties of the molecular sieve can be altered by means of cation exchange. At the same time, in the structure of the molecular sieve, there is an abundant pore channel system with a certain pore size, and these pore channels interlace with each other to form a three-dimensional network structure, and the framework can still exist stably after the water or organic matter in the pore channels is removed (US 4439409). It is based on the above structure that molecular sieves not only exhibit good catalytic activity for various organic reactions and excellent shape selectivity, but also can achieve good selectivity through modification (US 6162416, US 4954325, US 5362697).

As a novel molecular sieve, SCM-36 has the advantages of thin crystal thickness, large external specific surface area, large acid amount, fast molecular diffusion, simple preparation method, and high yield. Compared with fossil resources, biomass resources have a higher oxygen content. To obtain higher-value chemicals from biomass resources, it is necessary to involve deoxygenation and dehydration reactions. In the process of biomass conversion, in addition to the desired target product, different types of oligomers are often formed. For example, in the reaction of catalytic dehydration of hexose (glucose, fructose) to prepare 5-hydroxymethylfurfural (HMF), in addition to the main product HMF, complex polymerization reactions also occur between the starting materials, intermediates, and even the target product, generating oligomers of various molecular weights, and finally forming humins. In this regard, catalysts with a layered structure and a large external specific surface area are more conducive to product diffusion and reduce the formation of oligomers. Therefore, the structural characteristics of the SCM-36 molecular sieve give it a significant advantage in the dehydration reaction of hexoses.

5-Hydroxymethylfurfural (HMF) is an important furan derivative, known as a significant bridge connecting biomass chemical industry and petrochemical industry, and its synthesis has received extensive attention from researchers worldwide. Acidic catalysts are commonly used catalysts in biomass dehydration or etherification reactions, including inorganic acids, inorganic salts, solid acids, and the like. Solid acids have the advantages of no equipment corrosion, low pollution, and easy separation.

CN103028424A reports a method for preparing a solid acid catalyst for the synthesis of 5-hydroxymethylfurfural, comprising dissolving an oxide in deionized water, precipitating it completely with a precipitant, followed by aging, then washing, filtering, and drying, and then soaking the precipitate in a sulfuric acid solution or a chromium solution, and after impregnation, calcining to obtain a solid acid catalyst. The catalyst is used for the synthesis of 5-hydroxymethylfurfural with a high yield. However, since the main active components of the catalyst are sulfate ions and chromium ions, it imposes a certain burden on the environment.

CN101812039A provides a method for producing 5-hydroxymethylfurfural using an ionic liquid catalyst. The reaction belongs to a homogeneous catalytic reaction, with a small amount of catalyst added and a high yield. However, the reaction steps are complicated, requiring processes such as distillation, the selection of the types of cations and anions of the ionic liquid catalyst is complex, and the organic reagents also impose a certain burden on the environment.

In summary, the methods for preparing 5-hydroxymethylfurfural in the prior art have problems such as many by-products, low yield, high catalyst toxicity, and difficulty in recovery. Therefore, it is necessary to find a suitable reaction system to improve the yield of 5-hydroxymethylfurfural, reduce the formation of by-products, and improve the recovery and utilization rate of the catalyst to meet the needs of green production.

## SUMMARY

The technical problem to be solved by the present disclosure is the problems existing in the prior art for the

preparation process of 5-hydroxymethylfurfural (HMF) and/or 5-(alkoxymethyl)furfural (RMF), such as low catalytic efficiency, high cost, difficult product separation, and environmental pollution. To solve the above technical problems, the present disclosure provides a modified SCM-36 molecular sieve and a preparation method and use thereof. The catalyst has the advantages of good stability, simple preparation, high product selectivity, and easy separation from the product, achieving a green and efficient conversion of biomass to HMF and/or RMF.

In a first aspect, the present disclosure provides a modified SCM-36 molecular sieve having a schematic chemical composition represented by the formula "$mSiO_2{\bullet}nAl_2O_3{\bullet}pSO_3H$", wherein $22 \leq m/n \leq 80$, and $10 \leq m/p \leq 160$.

In some embodiments, the modified SCM-36 molecular sieve is obtained by subjecting a hydrogen-form SCM-36 molecular sieve to sulfonation modification, and the ratio of terminal silanol groups to the sum of internal silanol groups and bridging hydroxyl groups in the hydrogen-form SCM-36 molecular sieve is in a range of 0.2-1.2. In some embodiments, the Brønsted acid/Lewis acid ratio of the modified SCM-36 molecular sieve is in a range of 2.0-4.5, preferably 2.2-4.0. In some embodiments, in the SCM-36 molecular sieve, the content of $-SO_3H$ is in a range of 1.5 wt%-20.0 wt%, preferably 1.9 wt%-18.0 wt%. In some embodiments, the external specific surface area/total specific surface area ratio of the modified SCM-36 molecular sieve is in a range of 0.25-0.9, preferably 0.25-0.7. In some embodiments, the modified SCM-36 molecular sieve has a nanosheet-like morphology, with a thickness of the nanosheets not exceeding 11 nm, and a length and width each being in a range of 110-530 nm. In some embodiments, in the modified SCM-36 molecular sieve, the nanosheets with a thickness not exceeding 6 nm account for at least 65%, preferably at least 75%, of the total number of nanosheets counted.

In a second aspect, the present disclosure provides a method for preparing the above modified SCM-36 molecular sieve, comprising: subjecting a hydrogen-form SCM-36 molecular sieve to acid elution, addition of a mercapto compound, oxidation, and hydrothermal treatment.

In some embodiments, the method for preparing the modified SCM-36 molecular sieve comprises the following steps:

S1: mixing a silicon source, an aluminum source, an alkali source, an organic structure-directing agent, and water to obtain a mixture, then subjecting the mixture to crystallization, a first calcination, ion exchange, and a second calcination to obtain a hydrogen-form SCM-36 molecular sieve;
S2: dispersing the hydrogen-form SCM-36 molecular sieve in a solvent, adding a mercapto compound to form a reaction solution A; filtering the reaction solution A, followed by washing under reflux, to obtain a solid B;
S3: mixing the solid B, an oxidizing agent, an alcohol, and water to obtain a suspension; filtering the suspension, followed by post-treatment, to obtain a modified SCM-36-$SO_3H$ molecular sieve.

In a third aspect, the present disclosure also provides use of the modified SCM-36 molecular sieve as a solid acid catalyst, preferably use as a solid acid catalyst in a dehydration reaction, more preferably use as a solid acid catalyst in a dehydration reaction of a hexose-containing feedstock.

In a fourth aspect, the present disclosure also provides a method for dehydrating a hexose-containing feedstock, the method comprising: subjecting a hexose-containing feedstock to a dehydration reaction in the presence of a solid acid catalyst to produce 5-hydroxymethylfurfural and/or 5-(alkoxymethyl)furfural, wherein the solid acid catalyst is the modified SCM-36 molecular sieve as described above.

Compared with the prior art, the present disclosure has the following beneficial effects:

(1) In the present disclosure, an SCM series molecular sieve with a novel structure serves as a base material, and hydrogen-form SCM-36 having a relatively large number of silanol groups on the surface is used to carry out sulfonate loading, obtaining a modified SCM-36-$SO_3H$ molecular sieve material. SCM-36, as a novel molecular sieve, has the advantages of thin crystal thickness, large external specific surface area, large acid amount, fast molecular diffusion, simple preparation method, and high yield.

(2) In the dehydration reaction, particularly the biomass $\rightarrow$ HMF reaction, the use of modified SCM-36-$SO_3H$ molecular sieve as a solid acid catalyst provides the method with the advantages of simple operation, high product selectivity, good catalyst stability, easy product separation, and easy catalyst recovery, achieving a green and efficient conversion of biomass to HMF and/or RMF.

Additional features and advantages of the present application will be described in detail in the subsequent detailed description section.

## BRIEF DESCRIPTION OF THE DRAWINGS

FIG. 1 shows an X-ray diffraction pattern (XRD pattern) of an SCM-36 molecular sieve;
FIG. 2 shows infrared spectra of a hydrogen-form SCM-36 molecular sieve and a hydrogen-form C-FER molecular sieve;
FIG. 3 shows a pyridine adsorption infrared spectrum of a hydrogen-form SCM-36 molecular sieve;
FIG. 4 shows an XRD pattern of the SCM-36-$SO_3H$ molecular sieve obtained in Example 1;
FIG. 5 shows an SEM image of the SCM-36-$SO_3H$ molecular sieve obtained in Example 1;
FIG. 6 shows a pyridine adsorption infrared spectrum of the SCM-36-$SO_3H$ molecular sieve obtained in Example 1.

## DETAILED DESCRIPTION

The specific embodiments of the present application will be described in detail below with reference to the accompanying drawings. It should be understood that the specific embodiments described herein are only used to illustrate and explain the present application and are not intended to limit the present application.

Any specific numerical value (including the endpoints of numerical ranges) disclosed herein is not limited to the precise value but should be understood to also encompass values close to that precise value, such as all possible values within a range of $\pm$5% of that precise value. Furthermore, for disclosed numerical ranges, one or more new numerical ranges can be obtained by arbitrary combination between the endpoint values of the range, between endpoint values and specific point values within the range, and between various specific point values. These new numerical ranges should also be considered as specifically disclosed herein.

Unless otherwise indicated, the terms used herein have the same meanings as commonly understood by a person skilled in the art. If a term is defined herein and the definition differs from the common understanding in the art, the definition herein shall prevail.

As mentioned above, in a first aspect, the present disclosure provides a modified SCM-36 molecular sieve having a schematic chemical composition represented by the formula "$mSiO_2 \bullet nAl_2O_3 \bullet pSO_3H$", wherein $22 \leq m/n \leq 80$, and $10 \leq m/p \leq 160$. In some embodiments, in the schematic chemical composition of the modified SCM-36 molecular sieve, preferably $25 \leq m/n \leq 66$, $25 \leq m/p \leq 120$; more preferably $25 \leq m/n \leq 60$, $26 \leq m/p \leq 80$. For example, m/n may be 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or in a range between any two of the above values; for example, m/p may be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, or in a range between any two of the above values. In the present disclosure, the values of m/n and m/p can be obtained by measuring elemental contents using inductively coupled plasma atomic emission spectrometry and then calculating.

In some embodiments, the modified SCM-36 molecular sieve is obtained by subjecting a hydrogen-form SCM-36 molecular sieve to sulfonation modification, the silica-alumina ratio of the hydrogen-form SCM-36 molecular sieve is in a range of 20-50, for example, may be 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or in a range between any two of the above values; the ratio of terminal silanol groups to the sum of internal silanol groups and bridging hydroxyl groups in the hydrogen-form SCM-36 molecular sieve is in a range of 0.2-1.2, for example, may be 0.2, 0.3, 0.4, 0.5, 0.6, 0.7, 0.8, 0.9, 1.0, 1.1, 1.2, or in a range between any two of the above values.

The SCM-36 molecular sieve and the preparation method thereof are described in CN115959681A, the entire contents of which are incorporated herein by reference.

In some embodiments, the Brønsted acid/Lewis acid ratio of the modified SCM-36 molecular sieve is in a range of 2.0-4.5, preferably 2.2-4.0. In some embodiments, the Brønsted acid/Lewis acid ratio of the modified SCM-36 molecular sieve may be 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, or in a range between any two of the above values. In the present disclosure, the acid type and acid amount can be determined by pyridine adsorption infrared spectroscopy, and the Brønsted acid/Lewis acid ratio can be calculated.

In some embodiments, in the modified SCM-36 molecular sieve, the content of -$SO_3H$ is in a range of 1.5 wt%-20.0 wt%, preferably 1.9 wt%-18.0 wt%. In some embodiments, the content of -$SO_3H$ may be 1.5 wt%, 1.6 wt%, 1.7 wt%, 1.8 wt%, 1.9 wt%, 2.0 wt%, 2.5 wt%, 3.0 wt%, 3.5 wt%, 4.0 wt%, 4.5 wt%, 5.0 wt%, 5.5 wt%, 6.0 wt%, 6.5 wt%, 7.0 wt%, 7.5 wt%, 8.0 wt%, 8.5 wt%, 9.0 wt%, 9.5 wt%, 10.0 wt%, 10.5 wt%, 11.0 wt%, 11.5 wt%, 12.0 wt%, 12.5 wt%, 13.0 wt%, 13.5 wt%, 14.0 wt%, 14.5 wt%, 15.0 wt%, 15.5 wt%, 16.0 wt%, 16.5 wt%, 17.0 wt%, 17.5 wt%, 18.0 wt%, 18.5 wt%, 19.0 wt%, 19.5 wt%, 20.0 wt%, or in a range between any two of the above values. In the present disclosure, the content of -$SO_3H$ can be obtained by measuring the S element content using inductively coupled plasma atomic emission spectrometry and then calculating.

In some embodiments, the modified SCM-36 molecular sieve is an SCM-36 molecular sieve having -$SO_3H$ groups, wherein the SCM-36 molecular sieve has a schematic chemical composition represented by the formula "$mSiO_2 \cdot nAl_2O_3$", wherein $22 \leq m/n \leq 80$, preferably $25 \leq m/n \leq 66$, more preferably $25 \leq m/n \leq 60$, wherein the content of -$SO_3H$ groups is in a range of 1.5 wt%-20.0 wt%, preferably 1.9 wt%-18.0 wt%, based on the weight of the modified SCM-36 molecular sieve. In some embodiments, m/n may be 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, or in a range between any two of the above values; the content of -$SO_3H$ groups may be 1.5 wt%, 1.6 wt%, 1.7 wt%, 1.8 wt%, 1.9 wt%, 2.0 wt%, 2.5 wt%, 3.0 wt%, 3.5 wt%, 4.0 wt%, 4.5 wt%, 5.0 wt%, 5.5 wt%, 6.0 wt%, 6.5 wt%, 7.0 wt%, 7.5 wt%, 8.0 wt%, 8.5 wt%, 9.0 wt%, 9.5 wt%, 10.0 wt%, 10.5 wt%, 11.0 wt%, 11.5 wt%, 12.0 wt%, 12.5 wt%, 13.0 wt%, 13.5 wt%, 14.0 wt%, 14.5 wt%, 15.0 wt%, 15.5 wt%, 16.0 wt%, 16.5 wt%, 17.0 wt%, 17.5 wt%, 18.0 wt%, 18.5 wt%, 19.0 wt%, 19.5 wt%, 20.0 wt%, or in a range between any two of the above values, based on the weight of the modified SCM-36 molecular sieve. In the present disclosure, the value of m/n and the content of -$SO_3H$ groups can be obtained by measuring elemental contents using inductively coupled plasma atomic emission spectrometry and then calculating.

In some embodiments, the external specific surface area/total specific surface area ratio of the modified SCM-36 molecular sieve is in a range of 0.25-0.9, preferably 0.25-0.7. For example, the external specific surface area/total specific surface area ratio of the modified SCM-36 molecular sieve may be 0.25, 0.30, 0.35, 0.40, 0.45, 0.50, 0.55, 0.60, 0.65, 0.70, 0.75, 0.80, 0.85, 0.90, or in a range between any two of the above values. In the present disclosure, the specific surface area can be measured by the BET method, and then the external specific surface area/total specific surface area ratio can be obtained by calculation.

In some embodiments, the modified SCM-36 molecular sieve has a nanosheet-like morphology, with a thickness of the nanosheets not exceeding 11 nm, and a length and width each being in a range of 110-530 nm; preferably, the modified SCM-36 molecular sieve has a nanosheet-like morphology with a thickness not exceeding 10 nm, and a length and width each in a range of 120-430 nm. In some embodiments, in the modified SCM-36 molecular sieve, the nanosheets with a thickness not exceeding 6 nm account for at least 65%, preferably at least 75%, of the total number of nanosheets counted.

In a second aspect, the present disclosure provides a method for preparing the SCM-36-$SO_3H$ molecular sieve, comprising: subjecting a hydrogen-form SCM-36 molecular sieve to acid elution, addition of a mercapto compound, oxidation, and hydrothermal treatment. The hydrogen-form SCM-36 molecular sieve can be prepared according to the method described in CN115959681A.

In some embodiments, the method for preparing the SCM-36-$SO_3H$ molecular sieve comprises the following steps:

S1: mixing a silicon source, an aluminum source, an alkali source, an organic structure-directing agent, and water to obtain a mixture, then subjecting the mixture to crystallization, a first calcination, ion exchange, and a second calcination to obtain a hydrogen-form SCM-36 molecular sieve;

S2: dispersing the hydrogen-form SCM-36 molecular sieve in a solvent, adding a mercapto compound to form a reaction solution A; filtering the reaction solution A, followed by washing under reflux, to obtain a solid B;

S3: mixing the solid B, an oxidizing agent, an alcohol, and water to obtain a suspension; filtering the suspension, followed by post-treatment, to obtain a modified SCM-36-$SO_3H$ molecular sieve.

In some embodiments, the SCM-36 molecular sieve as-synthesized is calcined to remove the structure-directing agent, then subjected to ion exchange and calcination to obtain a hydrogen-form SCM-36 molecular sieve. Herein, calcination and ion exchange are conventional operations in the art. Preferably, the hydrogen-form SCM-36 molecular sieve has a silica-alumina ratio in a range of 20-50; the ratio of terminal silanol groups to the sum of internal silanol groups and bridging hydroxyl groups is in a range of 0.2-1.2.

In some embodiments, the type of silicon source is not particularly limited and can be conventional choices in the art. Preferably, the silicon source is at least one of silica sol and silica gel.

In some embodiments, the type of aluminum source is not particularly limited and can be conventional choices in the art. Preferably, the aluminum source is an aluminate, such as sodium aluminate.

In some embodiments, the type of alkali source is not particularly limited and can be conventional choices in the art. Preferably, the alkali source is sodium hydroxide.

In some embodiments, the organic structure-directing agent contains a trimethylethylammonium ion structure.

In some embodiments, the aluminum source has an $Al_2O_3$ content in a range of 38 wt%-43 wt%, and the alkali source has a $Na_2O$ content in a range of 30 wt%-33 wt%.

In some embodiments, in the mixture of step S1, the molar ratio of the silicon source (calculated as $SiO_2$), the aluminum source (calculated as $Al_2O_3$), the alkali source (calculated as $OH^-$), the organic structure-directing agent (calculated as trimethylethylammonium ion), and water is in a range of 1 : 0.016-0.050 : 0.10-0.20 : 0.15-0.30 : 10-50.

In some embodiments, the crystallization conditions for the mixture of step S1 include: crystallization temperature: 155-175°C, for example, the crystallization temperature may be 155°C, 160°C, 165°C, 170°C, 175°C, or in a range between any two of the above values; crystallization time: 2-8 d, for example, the crystallization time may be 2 d, 3 d, 4 d, 5 d, 6 d, 7 d, 8 d, or in a range between any two of the above values.

In some embodiments, step S1 further comprises washing and drying the mixture after the crystallization and before the first calcination. In some embodiments, the washing method can be conventional choices in the art, as long as the purpose of the present disclosure can be achieved. In some embodiments, the drying conditions can be conventional choices in the art, as long as the purpose of the present disclosure can be achieved. In the present disclosure, drying in an oven at 100°C overnight is exemplified to illustrate the advantages of the present disclosure.

In some embodiments, the conditions for the first calcination and the second calcination can be conventional choices in the art, as long as the purpose of the present disclosure can be achieved. In the present disclosure, calcining in air at 550°C for 6 h is exemplified for both the first calcination and the second calcination to illustrate the advantages of the present disclosure.

In some embodiments, the method of ion exchange is a conventional technical choice in the art, as long as the purpose of the present disclosure can be achieved. In the present disclosure, ammonium exchange is exemplified to illustrate the advantages of the present disclosure.

In some embodiments, in step S2, the solvent includes an inorganic solvent such as water; a neutral organic solvent such as toluene, benzene, n-hexane, etc.

In some embodiments, in step S2, the mercapto compound is selected from one or more of mercaptoethanol, thioglycolic acid, 3-mercaptopropyltrimethoxysilane, 3-mercaptopropyltriethoxysilane, dimethylsulfoniopropionate, methyl mercaptopropionate, 1,3-dimercaptopropane, or 2,3-dimercapto-1-propanol; preferably 3-mercaptopropyltrimethoxysilane or 3-mercaptopropyltriethoxysilane.

In some embodiments, in step S2, the mass ratio of the solvent to the molecular sieve is in a range of 10-200:1, preferably 20-100:1, for example, may be 10:1, 20:1, 30:1, 40:1, 50:1, 60:1, 70:1, 80:1, 90:1, 100:1, 110:1, 120:1, 130:1, 140:1, 150:1, 160:1, 170:1, 180:1, 190:1, 200:1, or in a range between any two of the above values. In some embodiments, the mass ratio of the mercapto compound to the hydrogen-form SCM-36 molecular sieve is in a range of 0.0001-0.5, preferably 0.0005-0.5, for example, may be 0.0001, 0.0005, 0.001, 0.005, 0.01, 0.05, 0.1, 0.2, 0.3, 0.4, 0.5, or in a range between any two of the above values.

In some embodiments, step S2 further comprises thoroughly stirring the reaction solution A before filtration, with conditions for stirring including: temperature in a range of 25-30°C, and time in a range of 5-48 h.

In some embodiments, in step S2, the method of washing under reflux is a conventional technical choice in the art, as long as the purpose of the present disclosure can be achieved. In the present disclosure, washing under reflux can be carried out by using dichloromethane and ethyl acetate. In some embodiments, in step S2, the mass ratio of dichloromethane to the hydrogen-form SCM-36 molecular sieve is in a range of 10-80:1, for example, may be 10:1, 20:1, 30:1, 40:1, 50:1, 60:1, 70:1, 80:1, or in a range between any two of the above values; the mass ratio of ethyl acetate to the hydrogen-form SCM-36 molecular sieve is in a range of 10-80:1, for example, may be 10:1, 20:1, 30:1, 40:1, 50:1, 60:1, 70:1, 80:1, or in a range between any two of the above values; the time for washing under reflux is in a range of 6-30 h, for example, may be 6 h, 8 h, 10 h, 12 h, 14 h, 16 h, 18 h, 20 h, 22 h, 24 h, 26 h, 28 h, 30 h, or in a range between any two of the above values.

In some embodiments, in step S3, the type of oxidizing agent is not particularly limited and can be conventional choices in the art. According to a preferable embodiment of the present disclosure, the oxidizing agent is hydrogen peroxide or oxygen.

In some embodiments, in step S3, the mass ratio of the oxidizing agent to the hydrogen-form SCM-36 molecular sieve is in a range of 1-100:1, preferably 2-60:1, for example, may be 1:1, 2:1, 3:1, 4:1, 5:1, 6:1, 7:1, 8:1, 9:1, 10:1, 20:1, 30:1, 40:1, 50:1, 60:1, 70:1, 80:1, 90:1, 100:1, or in a range between any two of the above values.

In some embodiments, in step S3, the alcohol is one or more of methanol or ethanol. The mass ratio of the alcohol to the hydrogen-form SCM-36 molecular sieve is in a range of 100-300:1, for example, may be 100:1, 110:1, 120:1, 130:1, 140:1, 150:1, 160:1, 170:1, 180:1, 190:1, 200:1, 210:1, 220:1, 230:1, 240:1, 250:1, 260:1, 270:1, 280:1, 290:1, 300:1, or in a range between any two of the above values.

In some embodiments, in step S3, the mass ratio of water to the hydrogen-form SCM-36 molecular sieve is in a range of 200-600:1, for example, may be 200:1, 250:1, 300:1, 350:1, 400:1, 450:1, 500:1, 550:1, 600:1, or in a range between any two of the above values.

In some embodiments, in step S3, mixing the solid B, oxidizing agent, alcohol, and water uniformly is achieved by stirring at room temperature, with conditions for stirring at room temperature including: temperature in a range of 25-30°C, and time in a range of 8-30 h.

In some embodiments, in step S3, the post-treatment after filtration includes adding dilute acid, stirring, filtering, washing, and drying. In some embodiments, the dilute acid is at least one of dilute hydrochloric acid, dilute sulfuric acid, and dilute nitric acid, preferably dilute sulfuric acid. In some embodiments, the concentration of the dilute acid is in a range

of 0.1-1.0 mol/L, and the amount of dilute acid added is in a range of 10-100 mL per 1 g of molecular sieve; the conditions for stirring in the dilute acid include: temperature in a range of 25-30°C, and time in a range of 5-48 h. The washing method can be conventional choices in the art, as long as the purpose of the present disclosure can be achieved, for example, washing can be performed with water, alcohol, or a mixture thereof. In some embodiments, the drying conditions can be conventional choices in the art, as long as the purpose of the present disclosure can be achieved.

In a third aspect, the present disclosure also provides use of the modified SCM-36 molecular sieve as a solid acid catalyst, preferably use as a solid acid catalyst in a dehydration reaction, more preferably use as a solid acid catalyst in a dehydration reaction of a hexose-containing feedstock.

In some embodiments, the dehydration reaction is a reaction of hexose dehydration to produce 5-hydroxymethylfurfural and/or 5-(alkoxymethyl)furfural. In other embodiments, the dehydration reaction is a reaction of 2,5-hexanedione dehydration to produce 2,5-dimethylfuran.

In a fourth aspect, the present disclosure also provides a method for dehydrating a hexose-containing feedstock, the method comprising: subjecting a hexose-containing feedstock to a dehydration reaction in the presence of a solid acid catalyst to produce 5-hydroxymethylfurfural and/or 5-(alkoxymethyl)furfural, wherein the solid acid catalyst is the modified SCM-36 molecular sieve according to the present disclosure.

In some embodiments, the hexose-containing feedstock is selected from one or more of galactose, mannose, glucose and fructose, preferably fructose and/or glucose, more preferably fructose.

In some embodiments, the 5-(alkoxymethyl)furfural is 5-(C1-C20 alkoxymethyl)furfural, preferably 5-(C1-C8 alkoxymethyl)furfural, more preferably 5-(C1-C5 alkoxymethyl)furfural.

In some embodiments, the conditions for the dehydration reaction include: a reaction temperature in a range of 80-200°C, preferably 100-180°C, for example, may be 80°C, 90°C, 100°C, 110°C, 120°C, 130°C, 140°C, 150°C, 160°C, 170°C, 180°C, 190°C, 200°C, or in a range between any two of the above values; a reaction time in a range of 5 min-20 h, preferably 10-200 min, for example, may be 5 min, 10 min, 30 min, 1 h, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, or in a range between any two of the above values.

In some embodiments, the dehydration reaction is carried out in at least one solvent. Preferably, the solvent is selected from at least one of water, alcohol, cyclic ether compounds, γ-valerolactone, methyl isobutyl ketone, acetone, toluene, dimethyl sulfoxide, N,N-dimethylformamide, and ionic liquids. Preferably, the alcohol contains 1 to 20 carbon atoms, preferably 1 to 8 carbon atoms, more preferably 1-5 carbon atoms, and is preferably selected from methanol, ethanol, n-propanol, n-butanol, isopropanol, isobutanol, isoamyl alcohol, ethylene glycol, propylene glycol, and glycerol; the cyclic ether compound is selected from pyran, 2-methyltetrahydrofuran, 2,5-dimethyltetrahydrofuran, tetrahydrofuran, and 1,4-dioxane; the ionic liquid is selected from imidazolium-based ionic liquids and pyridinium-based ionic liquids.

In some embodiments, the solvent comprises solvent I and solvent II, the solvent I is selected from at least one of water and alcohol, preferably, the alcohol contains 1 to 20 carbon atoms, preferably 1 to 8 carbon atoms, more preferably 1-5 carbon atoms, and is preferably selected from methanol, ethanol, n-propanol, n-butanol, isopropanol, isobutanol, isoamyl alcohol, ethylene glycol, propylene glycol, and glycerol; the solvent II is selected from at least one of cyclic ether compounds, methyl isobutyl ketone, and γ-valerolactone, the cyclic ether compound is preferably selected from at least one of pyran, 2-methyltetrahydrofuran, 2,5-dimethyltetrahydrofuran, tetrahydrofuran, and 1,4-dioxane, and the solvent II is preferably selected from one or two of 2,5-dimethyltetrahydrofuran, 1,4-dioxane, and tetrahydrofuran. Preferably, the mass ratio of the solvent I to the solvent II is in a range of 0.05-20:1, preferably 0.1-10:1.

In some embodiments, the mass ratio of the total amount of the solvent to the hexose-containing feedstock is in a range of 1-100:1, preferably 2-50:1, for example, may be 1:1, 2:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1, 100:1, or in a range between any two of the above values; the mass ratio of the hexose-containing feedstock to the catalyst is in a range of 0.2-20:1, preferably 0.4-20:1, for example, may be 0.2:1, 0.3:1, 0.4:1, 0.5:1, 0.6:1, 0.7:1, 0.8:1, 0.9:1, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, or in a range between any two of the above values.

In some embodiments, the dehydration reaction is carried out under an inert gas (such as nitrogen and/or argon), and the inert gas pressure is in a range of 0.1-3.0 MPa, preferably 0.5-2.0 MPa, for example, may be 0.1 MPa, 0.2 MPa, 0.4 MPa, 0.6 MPa, 0.8 MPa, 1 MPa, 1.5 MPa, 2 MPa, 2.5 MPa, 3.0 MPa, or in a range between any two of the above values.

In the present disclosure, pressure refers to gauge pressure, unless otherwise specified.

In the present disclosure, room temperature refers to a temperature of about 25°C, unless otherwise specified.

In the present disclosure, the XRD measurement method included: using a Rigaku (Japan) Ultima IV X-ray powder diffractometer, CuKα radiation source (λ = 1.54 Å), nickel filter, 2θ scanning range 2°-50°/75°, operating voltage 35 kV, current 25 mA, scanning rate 10°/min.

In the present disclosure, the acid amount and acid type of the catalyst were determined by pyridine adsorption infrared spectroscopy. The instrument used was a Nicolet Model 710 spectrometer. The specific operation steps were as follows: a. Sample pretreatment: the sample (about 30 mg) was pressed into a thin circular disk with a diameter of 13 mm and placed in an infrared sample cell; then, the sample was pretreated at 400°C for 1 h under vacuum cell conditions; after the sample cell was cooled to room temperature, the infrared data of the sample was scanned as background; b. Pyridine

adsorption: at room temperature and under vacuum environment, pyridine vapor was introduced into the in-situ cell until adsorption reached equilibrium, the adsorption time was 1 h, and then the infrared absorption spectrum was scanned and recorded; c. Pyridine desorption: after adsorption, vacuum was applied at 100°C until the internal pressure no longer changed (about $10^{-3}$ Pa), the desorption time was 40 min, and then the infrared absorption spectrum was scanned and recorded. The difference spectrum before and after pyridine adsorption was the obtained pyridine adsorption-infrared absorption spectrum. According to the spectrum, the acid amount of the sample was calculated using the following formulas:

$$C_L = K_L \times A_{1440} = \frac{\pi}{IMEC_L} \times \left(\frac{r^2}{w}\right) \times A_{1440}$$

$$C_B = K_B \times A_{1540} = \frac{\pi}{IMEC_B} \times \left(\frac{r^2}{w}\right) \times A_{1540}$$

wherein, r and w are the diameter (cm) and mass (g) of the thin circular disk of the catalyst, respectively, and A is the integrated absorbance value of the peak at the specified wavenumber according to the scanned pyridine adsorption-infrared absorption spectrum. IMEC is the integrated molar extinction coefficient, wherein $IMEC_L$ is 2.22 and $IMEC_B$ is 1.67, $C_L$ is the Lewis acid amount ($\mu mol \cdot g^{-1}$), and $C_B$ is the Brønsted acid amount ($\mu mol \cdot g^{-1}$).

In the present disclosure, the model of the scanning electron microscope (SEM) used was Hitachi S-4800II field emission scanning electron microscope.

In the present disclosure, the specific surface area and external specific surface area of the molecular sieve were measured by the nitrogen physical adsorption-desorption method (BET method). The nitrogen physical adsorption-desorption isotherms of the molecular sieve were measured using a physical adsorption instrument (Micromeritics ASAP 2020M). The obtained results were analyzed by the Brunauer-Emmet-Teller (BET) method to obtain the specific surface area of the sample, and the pore volume and pore size distribution of the sample were analyzed by the Barrett-Joyner-Halenda (BJH) method. Experimental conditions: the sample was vacuum degassed at 350°C for 4 h before analysis to remove moisture and volatile impurities, and then the nitrogen adsorption-desorption test was performed in a liquid nitrogen environment (-196°C).

In the present disclosure, the model of the inductively coupled plasma atomic emission spectrometer (ICP) used was Varian 725-ES. The sample to be analyzed was dissolved with hydrofluoric acid to detect the content of elements.

In the present disclosure, 5-hydroxymethylfurfural (HMF) and 5-(alkoxymethyl)furfural (RMF) were qualitatively analyzed by gas chromatography-mass spectrometry (GC-MS). The conversion of hexose and the yields of 5-hydroxymethylfurfural and 5-(alkoxymethyl)furfural were analyzed by gas chromatography (GC) and high-performance liquid chromatography (HPLC). The gas chromatography-mass spectrometer was an Agilent (USA) G7077BA\MSD, with an INNOWAX gas chromatography capillary column (60 m, 0.32 mm); the gas chromatograph was an Agilent 8860, the detector was a flame ionization detector (FID), with an INNOWAX gas chromatography capillary column (60 m, 0.32 mm). The high-performance liquid chromatograph model was Agilent 1260 Prime II, and signal detection was performed by a refractive index detector (RID). The chromatographic column used included a Hi-Plex H column, with dilute sulfuric acid as the mobile phase, a flow rate of 0.6 mL/min, and a column temperature of 60°C.

The calculation formula for the yield of the product HMF and/or RMF is:

Yield of HMF and/or RMF % = (moles of HMF and/or RMF produced by the reaction, $n_1$) / (moles of hexose units in the reaction substrate biomass, $n_0$) $\times$ 100%;

wherein, the chemical formula of hexose is $C_6H_{12}O_6$.

To facilitate understanding of the present disclosure, the present disclosure provides examples as follows. However, these examples are only for helping to understand the present disclosure and should not be considered as specific limitations on the present disclosure.

## Examples

Unless otherwise specified, the experimental methods used in the following examples and comparative examples were conventional methods. Unless otherwise specified, the materials, reagents, etc., used in the following examples and comparative examples were commercially available, with a purity of analytical grade.

**Example 1 (Synthesis of SCM-36-$SO_3H$)**

14.35 g of deionized water, 0.604 g of sodium aluminate (containing 40.5 wt% $Al_2O_3$, 30.6 wt% $Na_2O$, balance water), 0.366 g of sodium hydroxide, 7.06 g of trimethylethylammonium hydroxide solution (containing 25.00 wt% trimethylethylammonium hydroxide, balance water) (organic structure-directing agent), and 12.60 g of silica sol (containing 40.0 wt% $SiO_2$, balance water) were mixed and stirred at room temperature for 4 h to obtain a mixture. The final material ratio (molar ratio) was:

$SiO_2/Al_2O_3$ = 35;
$OH^-/SiO_2$ = 0.18;
Trimethylethylammonium hydroxide (R)/$SiO_2$ = 0.20;
$H_2O/SiO_2$ = 18.

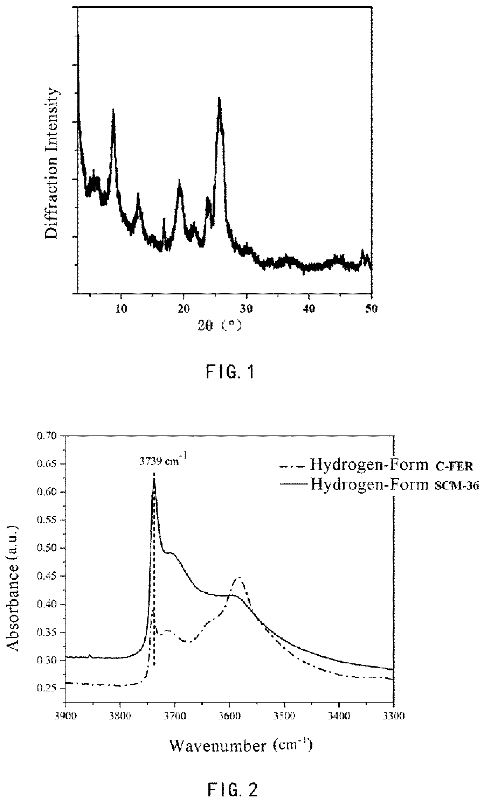

The mixture was charged into a stainless steel autoclave and crystallized by heating at 170°C with a rotation speed of 20 rpm for 3 days. After crystallization, the mixture was filtered, washed, and dried in an oven at 100°C overnight to obtain a product. The XRD pattern of the obtained product is shown in FIG. 1, which was SCM-36 molecular sieve.

The obtained SCM-36 molecular sieve was calcined to remove the organic structure-directing agent, then subjected to ion exchange and calcination to obtain hydrogen-form SCM-36. The ratio of terminal silanol groups to the sum of internal silanol groups and bridging hydroxyl groups in the hydrogen-form SCM-36 molecular sieve was 0.38.

C-FER was synthesized following the method for SCM-36, but replacing trimethylethylammonium hydroxide with cyclohexylamine, keeping other conditions unchanged. The product was calcined to remove the organic structure-directing agent, then subjected to ion exchange and calcination to obtain hydrogen-form C-FER.

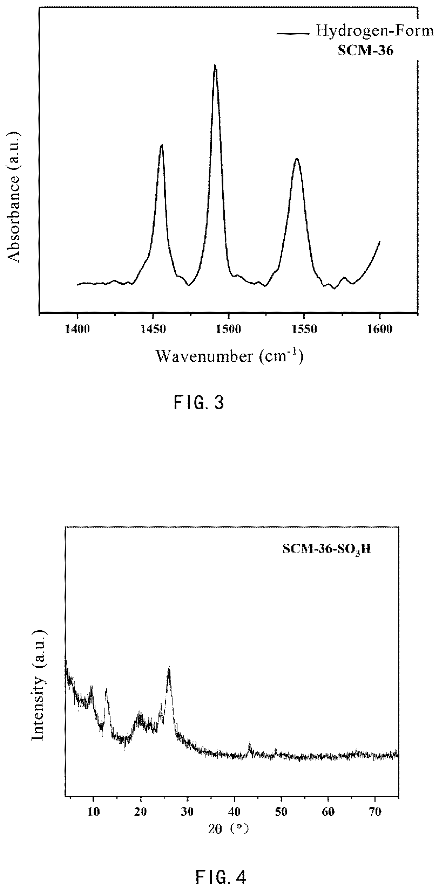

The infrared spectrum of hydrogen-form SCM-36 is shown in FIG. 2. The peak at 3739 $cm^{-1}$ is assigned to the surface silanol groups Si-OH of the molecular sieve. The figure shows that the hydrogen-form SCM-36 molecular sieve has more Si-OH than the hydrogen-form C-FER molecular sieve. The pyridine adsorption infrared spectrum of the hydrogen-form SCM-36 molecular sieve is shown in FIG. 3, and the Brønsted/Lewis acid ratio calculated therefrom was 0.9.

1.5 g of hydrogen-form SCM-36 molecular sieve and 1.0 g of 3-mercaptopropyltrimethoxysilane were added to 50 g of toluene, and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

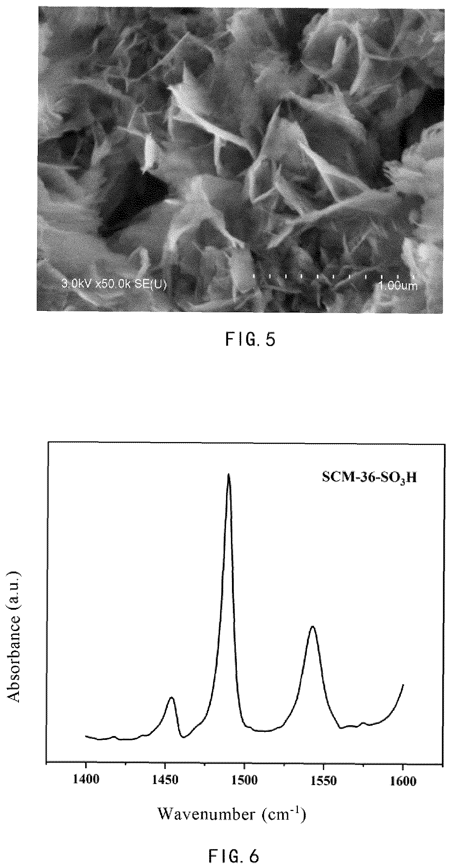

The XRD pattern of the SCM-36-$SO_3H$ molecular sieve is shown in FIG. 4, the SEM image is shown in FIG. 5, and the pyridine adsorption infrared spectrum is shown in FIG. 6, and the Brønsted/Lewis acid ratio calculated therefrom was 3.3. The -$SO_3H$ content of the SCM-36-$SO_3H$ molecular sieve, calculated from the S content determined by ICP, was 6.5 wt%, and the external specific surface area/total specific surface area ratio measured by BET was 0.55.

**Example 2**

The hydrogen-form SCM-36 molecular sieve was the same as in Example 1.

1.5 g of hydrogen-form SCM-36 and 0.7 g of 3-mercaptopropyltrimethoxysilane were added to 50 g of toluene, and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

The XRD pattern, SEM image, and pyridine adsorption infrared spectrum of the sample were similar to those in FIGS. 4-6. The Brønsted/Lewis acid ratio calculated from the pyridine adsorption infrared spectrum of the sample was 3.0. The -$SO_3H$ content of the sample, calculated from the S content determined by ICP, was 5.3 wt%, and the external specific surface area/total specific surface area ratio measured by BET was 0.53.

**Example 3**

The hydrogen-form SCM-36 molecular sieve was the same as in Example 1.

1.5 g of hydrogen-form SCM-36 and 1.2 g of 3-mercaptopropyltriethoxysilane were added to 50 g of toluene, and

the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

The XRD pattern, SEM image, and pyridine adsorption infrared spectrum of the sample were similar to those in FIGS. 4-6. The Brønsted/Lewis acid ratio calculated from the pyridine adsorption infrared spectrum of the sample was 3.1. The -$SO_3H$ content of the sample, calculated from the S content determined by ICP, was 5.5 wt%, and the external specific surface area/total specific surface area ratio measured by BET was 0.52.

**Example 4**

The hydrogen-form SCM-36 molecular sieve was the same as in Example 1.

1.5 g of hydrogen-form SCM-36 and 1.0 g of 3-mercaptopropyltrimethoxysilane were added to 50 g of toluene, and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 20 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

The XRD pattern, SEM image, and pyridine adsorption infrared spectrum of the sample were similar to those in FIGS. 4-6. The Brønsted/Lewis acid ratio calculated from the pyridine adsorption infrared spectrum of the sample was 2.9. The -$SO_3H$ content of the sample, calculated from the S content determined by ICP, was 5.1 wt%, and the external specific surface area/total specific surface area ratio measured by BET was 0.48.

**Example 5**

The hydrogen-form SCM-36 molecular sieve was the same as in Example 1.

1.5 g of hydrogen-form SCM-36 and 1.0 g of mercaptoethanol were added to 50 g of toluene, and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain SCM-36-$SO_3H$ molecular sieve.

The XRD pattern, SEM image, and pyridine adsorption infrared spectrum of the sample were similar to those in FIGS. 4-6. The Brønsted/Lewis acid ratio calculated from the pyridine adsorption infrared spectrum of the sample was 2.8. The -$SO_3H$ content of the sample, calculated from the S content determined by ICP, was 4.9 wt%, and the external specific surface area/total specific surface area ratio measured by BET was 0.51.

Table 1. Properties of the solid acid catalysts of Examples 1-5

| Catalyst No. | Brønsted/Lewis | -$SO_3H$ Content (wt%) | External Specific Surface Area/Total Specific Surface Area |
|---|---|---|---|
| Example 1 | 3.3 | 6.5 | 0.55 |
| Example 2 | 3.0 | 5.3 | 0.53 |
| Example 3 | 3.1 | 5.5 | 0.52 |
| Example 4 | 2.9 | 5.1 | 0.48 |
| Example 5 | 2.8 | 4.9 | 0.51 |

**Examples 6-10**

Fructose was used as a substrate; 0.2 g of the SCM-36-$SO_3H$ from Examples 1-5 above (corresponding to Examples 6-10, respectively), 0.5 g of fructose, 18 g of tetrahydrofuran (THF), and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer.

The reaction was carried out at 155°C for 25 min. The reaction product liquid was analyzed to obtain the fructose conversion and the yield of the target product 5-hydroxymethylfurfural (HMF), which are shown in Table 2.

**Example 11**

Glucose was used as a substrate; 0.2 g of the SCM-36-$SO_3H$ from Example 1 above, 0.5 g of glucose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a glucose conversion of 99.8% and a yield of the target product 5-hydroxymethylfurfural (HMF) of 45.3%.

**Example 12**

Fructose was used as a substrate; 0.5 g of the SCM-36-$SO_3H$ from Example 1 above, 3.5 g of fructose, 10 g of n-butanol, and 10 g of tetrahydrofuran were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a fructose conversion of 99.9%, a 5-hydroxymethylfurfural (HMF) yield of 30.5%, and a 5-butoxymethylfurfural (RMF) yield of 35.7%.

**Example 13**

Fructose was used as a substrate; 0.5 g of the SCM-36-$SO_3H$ from Example 1 above, 3.5 g of fructose, 10 g of ethanol, and 10 g of tetrahydrofuran were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a fructose conversion of 99.9%, a 5-hydroxymethylfurfural (HMF) yield of 32.8%, and a 5-ethoxymethylfurfural (RMF) yield of 39.6%.

**Comparative Example 1**

MCM-22 molecular sieve as-synthesized was prepared according to the method described in the literature [Chinese Journal of Catalysis 41 (2020) 1062-1066], calcined to remove the structure-directing agent, then subjected to ion exchange and calcination to obtain hydrogen-form MCM-22.

The hydrogen-form MCM-22 was modified according to Example 1 to obtain a modified MCM-22-$SO_3H$ molecular sieve.

The external specific surface area/total specific surface area ratio of the modified MCM-22-$SO_3H$ molecular sieve measured by BET was 0.12. The -$SO_3H$ content, calculated from the S content determined by ICP, was 3.5 wt%. The Brønsted/Lewis acid ratio calculated from the pyridine adsorption infrared spectrum was 1.2.

Fructose was used as a substrate, and THF and deionized water were used as solvents; 0.2 g of the above modified MCM-22-$SO_3H$, 0.5 g of fructose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a fructose conversion of 87.1% and a yield of the target product 5-hydroxymethylfurfural (HMF) of 30.2%.

**Comparative Example 2**

A commercially available ZSM-5 molecular sieve (Tianjin Nanhua Catalyst Co., Ltd., NKF-5D-38H) was acquired and modified according to Example 1 to obtain a modified ZSM-5-$SO_3H$ molecular sieve.

The external specific surface area/total specific surface area ratio of the modified ZSM-5-$SO_3H$ molecular sieve measured by BET was 0.06. The -$SO_3H$ content, calculated from the S content determined by ICP, was 0.5 wt%. The Brønsted/Lewis acid ratio calculated from the pyridine adsorption infrared spectrum was 1.1.

Fructose was used as a substrate, and THF and deionized water were used as solvents; 0.2 g of the above modified ZSM-5-$SO_3H$, 0.5 g of fructose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was

carried out at 155°C for 25 min. The reaction product liquid was analyzed to obtain a fructose conversion of 80.1% and a yield of the target product 5-hydroxymethylfurfural (HMF) of 15.3%.

**Comparative Example 3**

The hydrogen-form SCM-36 molecular sieve was the same as in Example 1.

Fructose was used as a substrate, and THF and deionized water were used as solvents; 0.2 g of the above hydrogen-form SCM-36, 0.5 g of fructose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a fructose conversion of 85.9% and a yield of the target product 5-hydroxymethylfurfural (HMF) of 36.8%.

**Comparative Example 4**

A commercially available A-15 ion exchange resin (Macklin Amberlyst 15 ion exchange resin, A800632) was acquired.

Glucose was used as a substrate; 0.2 g of A-15, 0.5 g of glucose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a glucose conversion of 99.8% and a yield of the target product 5-hydroxymethylfurfural (HMF) of 35.8%.

**Comparative Example 5**

The hydrogen-form SCM-36 molecular sieve was the same as in Example 1.

1.5 g of hydrogen-form SCM-36 and 0.2 g of 3-mercaptopropyltrimethoxysilane were added to 50 g of toluene, and the mixture was stirred thoroughly at 25-30°C for 24 h. After stirring, the mixture was filtered, and 30.0 g of dichloromethane and 30.0 g of ethyl acetate were added to wash under reflux for 24 h. Subsequently, the obtained solid was added to a mixed solution of 40 g of $H_2O_2$ (30%), 120 g of methanol, and 400 g of deionized water, and the mixture was stirred at room temperature for 15 h. After stirring, the mixture was filtered, and the precipitate was dispersed in 40 mL of sulfuric acid (0.6 mol/L) and stirred thoroughly for 15 h. Finally, the product was thoroughly washed with ethanol and water and dried to obtain an SCM-36-$SO_3H$ molecular sieve.

The external specific surface area/total specific surface area ratio of this SCM-36-$SO_3H$ molecular sieve measured by BET was 0.48. The -$SO_3H$ content, calculated from the S content determined by ICP, was 0.15 wt%. The Brønsted/Lewis acid ratio calculated from the pyridine adsorption infrared spectrum was 1.0.

Fructose was used as a substrate, and THF and deionized water were used as solvents; 0.2 g of the above hydrogen-form SCM-36, 0.5 g of fructose, 18 g of THF, and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain a fructose conversion of 86.7% and a yield of the target product 5-hydroxymethylfurfural (HMF) of 39.8%.

Table 2. Catalytic performance of Examples 6-13 and Comparative Examples 1-5

| Ex. | Biomass Feedstock | Solid Acid Catalyst | Solvent (mass ratio) | Mass Ratio of Feedstock/Catalyst | Feedstock Conversion (%) | HMF/RMF Yield (%) |
|---|---|---|---|---|---|---|
| 6 | Fructose | Example 1 | THF + Water (9:1) | 2.5 | 99.9 | 87.8 |
| 7 | Fructose | Example 2 | THF + Water (9:1) | 2.5 | 99.9 | 86.7 |
| 8 | Fructose | Example 3 | THF + Water (9:1) | 2.5 | 99.8 | 87.1 |
| 9 | Fructose | Example 4 | THF + Water (9:1) | 2.5 | 99.6 | 85.3 |

(continued)

| Ex. | Biomass Feedstock | Solid Acid Catalyst | Solvent (mass ratio) | Mass Ratio of Feedstock/Cat alyst | Feedstock Conversion (%) | HMF/RMF Yield (%) |
|---|---|---|---|---|---|---|
| 10 | Fructose | Example 5 | THF + Water (9:1) | 2.5 | 99.9 | 83.2 |
| 11 | Glucose | Example 1 | THF + Water (9:1) | 2.5 | 99.8 | 45.3 |
| 12 | Fructose | Example 1 | n-Butanol + THF (1:1) | 7 | 99.9 | 30.5(HMF) + 35.7(RMF) |
| 13 | Fructose | Example 1 | Ethanol + THF (1:1) | 7 | 99.9 | 32.8(HMF) + 39.6(RMF) |
| Comp. Ex. 1 | Fructose | MCM-22-$SO_3H$ | THF + Water (9:1) | 2.5 | 87.1 | 30.2 |
| Comp. Ex. 2 | Fructose | ZSM-5-$SO_3H$ | THF + Water (9:1) | 2.5 | 80.1 | 15.3 |
| Comp. Ex. 3 | Fructose | Hydrogen-form SCM-36 | THF + Water (9:1) | 2.5 | 85.9 | 36.8 |
| Comp. Ex. 4 | Glucose | A-15 Resin | THF + Water (9:1) | 2.5 | 99.8 | 35.8 |
| Comp. Ex. 5 | Fructose | SCM-36-$SO_3H$ (Non-inventive) | THF + Water (9:1) | 2.5 | 86.7 | 39.8 |

**Example 14**

Fructose was used as a substrate; 0.2 g of the catalyst from Example 1 above (SCM-36-$SO_3H$), 0.5 g of fructose, 18 g of tetrahydrofuran (THF), and 2 g of deionized water were added to an autoclave with stirrer. 0.5 MPa of nitrogen was charged to prevent the organic solvent from boiling. The temperature was raised to the preset temperature using a programmed heating mantle, and stirring was performed with a magnetic stirrer. The reaction was carried out at 155°C for 25 min. The reaction product liquid was analyzed to obtain the fructose conversion and the yield of the target product 5-hydroxymethylfurfural (HMF) for the 1st cycle. The used catalyst was recovered by filtration, washed, dried, and used in the next cycle. A total of 4 cycles of reaction were performed. The catalytic evaluation results are shown in Table 3.

Table 3. Recycling data for Catalyst of Example 1

| Number of Recycles | Fructose Conversion (%) | HMF Yield (%) |
|---|---|---|
| 1 | > 99 | 87.8 |
| 2 | > 99 | 85.1 |
| 3 | > 99 | 84.9 |
| 4 | > 99 | 84.3 |

**Example 15**

2,5-hexanedione was used as a substrate; 0.3 g of the SCM-36-$SO_3H$ from Example 1 above, 1.0 g of 2,5-hexanedione, 10 g of methyl isobutyl ketone, and 2.0 g of saturated sodium chloride aqueous solution were added to an autoclave with stirrer. 1 MPa of nitrogen was charged to prevent the organic solvent from boiling. The temperature was raised to the preset temperature using a programmed heating mantle, and stirring was performed with a magnetic stirrer. The reaction was carried out at 165°C for 8 h. The reaction product liquid was analyzed to obtain a 2,5-hexanedione conversion of 89.7% and a yield of the target product 2,5-dimethylfuran of 83.6%.

The specific embodiments of the present disclosure have been described in detail above, however, the present disclosure is not limited thereto. Within the technical concept of the present disclosure, various simple modifications can be made to the technical solution of the present disclosure, including combining various technical features in any other

suitable manner. These simple modifications and combinations should also be regarded as the content disclosed in the present disclosure and fall within the protection scope of the present disclosure.

## Claims

1. A modified SCM-36 molecular sieve, **characterized in that** the modified SCM-36 molecular sieve has a schematic chemical composition represented by the formula "$mSiO_2 \cdot nAl_2O_3 \cdot pSO_3H$", wherein $22 \leq m/n \leq 80$, and $10 \leq m/p \leq 160$.

2. The modified SCM-36 molecular sieve according to claim 1, wherein, in the schematic chemical composition of the modified SCM-36 molecular sieve, $25 \leq m/n \leq 66$, $25 \leq m/p \leq 120$, preferably $25 \leq m/n \leq 60$, $26 \leq m/p \leq 80$.

3. The modified SCM-36 molecular sieve according to claim 1 or 2, which is obtained by subjecting a hydrogen-form SCM-36 molecular sieve to sulfonation modification, preferably the ratio of terminal silanol groups to the sum of internal silanol groups and bridging hydroxyl groups in the hydrogen-form SCM-36 molecular sieve is in a range of 0.2-1.2.

4. The modified SCM-36 molecular sieve according to any one of claims 1-3, wherein the Bronsted acid/Lewis acid ratio of the modified SCM-36 molecular sieve is in a range of 2.0-4.5, preferably 2.2-4.0; and/or in the SCM-36-$SO_3H$ molecular sieve, the content of -$SO_3H$ is in a range of 1.5 wt%-20.0 wt%, preferably 1.9 wt%-18.0 wt%.

5. The modified SCM-36 molecular sieve according to any one of claims 1-4, wherein the external specific surface area/total specific surface area ratio of the modified SCM-36 molecular sieve is in a range of 0.25-0.9, preferably 0.25-0.7.

6. The modified SCM-36 molecular sieve according to any one of claims 1-5, wherein the modified SCM-36 molecular sieve has a nanosheet-like morphology, with a thickness of the nanosheets not exceeding 11 nm, and - length and width each being in a range of 110-530 nm; preferably, the modified SCM-36 molecular sieve has a nanosheet-like morphology with a thickness not exceeding 10 nm, and a length and width each in a range of 120-430 nm; and/or in the modified SCM-36 molecular sieve, the nanosheets with a thickness not exceeding 6 nm account for at least 65%, preferably at least 75%, of the total number of nanosheets counted.

7. A method for preparing a modified SCM-36 molecular sieve, **characterized in that** the preparation method comprises: subjecting a hydrogen-form SCM-36 molecular sieve to acid elution, addition of a mercapto compound, oxidation, and hydrothermal treatment.

8. The method for preparing a modified SCM-36 molecular sieve according to claim 7, wherein the preparation method comprises:

   S1: mixing a silicon source, an aluminum source, an alkali source, an organic structure-directing agent, and water to obtain a mixture, then subjecting the mixture to crystallization, a first calcination, ion exchange, and a second calcination to obtain a hydrogen-form SCM-36 molecular sieve;
   S2: dispersing the hydrogen-form SCM-36 molecular sieve in a solvent, adding a mercapto compound to form a reaction solution A; filtering the reaction solution A, followed by washing under reflux, to obtain a solid B;
   S3: mixing the solid B, an oxidizing agent, an alcohol, and water to obtain a suspension; filtering the suspension, followed by post-treatment, to obtain a modified SCM-36-$SO_3H$ molecular sieve.

9. The preparation method according to claim 8, wherein, in step S1,

   the solvent includes water and/or a neutral organic solvent, the neutral organic solvent is preferably selected from toluene, benzene, n-hexane; and/or
   the molar ratio of the silicon source calculated as $SiO_2$, the aluminum source calculated as $Al_2O_3$, the alkali source calculated as $OH^-$, the organic structure-directing agent, and water is in a range of 1 : 0.016-0.050 : 0.10-0.20 : 0.15-0.30 : 10-50; and/or
   the crystallization for the mixture is carried out at 150-180°C for 2-8 days.

10. The preparation method according to claim 8 or 9, wherein, in step S2,

the mass ratio of the solvent to the molecular sieve is in a range of 10-200:1, preferably 20-100:1; and/or
the mercapto compound is selected from at least one of mercaptoethanol, thioglycolic acid, 3-mercaptopropyltrimethoxysilane, 3-mercaptopropyltriethoxysilane, dimethylsulfoniopropionate, methyl mercaptopropionate, 1,3-dimercaptopropane, 2,3-dimercapto-1-propanol, preferably at least one of mercaptoethanol, thioglycolic acid, 3-mercaptopropyltrimethoxysilane, 3-mercaptopropyltriethoxysilane; and/or
the mass ratio of the mercapto compound to the hydrogen-form SCM-36 molecular sieve is in a range of 0.0001-0.5, preferably 0.0005-0.5; the stirring conditions include: temperature in a range of 25-30°C, and time in a range of 5-48 h; and/or
in step S2, the washing under reflux is carried out by using dichloromethane and ethyl acetate, wherein the mass ratio of dichloromethane to the hydrogen-form SCM-36 molecular sieve is in a range of 10-80:1; the mass ratio of ethyl acetate to the hydrogen-form SCM-36 molecular sieve is in a range of 10-80:1; the time for washing under reflux is in a range of 6-30 h; and/or
in step S3, the oxidizing agent is $H_2O_2$; and/or
the mass ratio of the oxidizing agent to the hydrogen-form SCM-36 molecular sieve is in a range of 1-100:1, preferably 2-60:1; and/or
the alcohol is one or more of methanol or ethanol; the mass ratio of the alcohol to the hydrogen-form SCM-36 molecular sieve is in a range of 100-300: 1; the mass ratio of water to the hydrogen-form SCM-36 molecular sieve is in a range of 200-600: 1; and/or
the post-treatment after filtration includes adding dilute acid, stirring, filtering, washing, and drying.

**11.** Use of the modified SCM-36 molecular sieve according to any one of claims 1-6 as a solid acid catalyst, preferably use as a solid acid catalyst in a dehydration reaction, more preferably use as a solid acid catalyst in a dehydration reaction of a hexose-containing feedstock.

**12.** A method for dehydrating a hexose-containing feedstock, **characterized in that** the method comprises: subjecting a hexose-containing feedstock to a dehydration reaction in the presence of a solid acid catalyst to produce 5-hydroxymethylfurfural and/or 5-(alkoxymethyl)furfural, wherein the solid acid catalyst comprises the modified SCM-36 molecular sieve according to any one of claims 1-6.

**13.** The method according to claim 12, wherein

the hexose-containing feedstock is selected from one or more of galactose, mannose, glucose and fructose; and/or
the conditions for the dehydration reaction include: a reaction temperature in a range of 80-200°C, preferably 100-180°C; a reaction time in a range of 5 min-20 h, preferably 10-200 min.

**14.** The method according to claim 12 or 13, wherein the dehydration reaction is carried out in at least one solvent; preferably, the solvent is selected from at least one of water, alcohol, cyclic ether compounds, γ-valerolactone, methyl isobutyl ketone, acetone, toluene, dimethyl sulfoxide, N,N-dimethylformamide, and ionic liquids; preferably, the alcohol contains 1 to 20 carbon atoms, preferably 1 to 8 carbon atoms, more preferably 1-5 carbon atoms, and is preferably selected from methanol, ethanol, n-propanol, n-butanol, isopropanol, isobutanol, isoamyl alcohol, ethylene glycol, propylene glycol, and glycerol; preferably, the cyclic ether compound is selected from pyran, 2-methyltetrahydrofuran, 2,5-dimethyltetrahydrofuran, tetrahydrofuran, and 1,4-dioxane; and/or,

the mass ratio of the total amount of the solvent to the hexose-containing feedstock is in a range of 1-100:1, preferably 2-50:1; and/or,
the mass ratio of the hexose-containing feedstock to the catalyst is in a range of 0.2-20:1, preferably 0.4-20:1.

**15.** The method according to any one of claims 12-14, wherein the dehydration reaction is carried out under an inert gas, and the inert gas pressure is in a range of 0.1-3.0 MPa, preferably 0.5-2.0 MPa.

Diffraction Intensity
10
20
30
40
50
2θ (°)

FIG. 1

0.70
0.65
0.60
0.55
0.50
0.45
0.40
0.35
0.30
0.25
3739 cm-1
Hydrogen-Form C-FER
Hydrogen-Form SCM-36
Absorbance (a.u.)
3900
3800
3700
3600
3500
3400
3300
Wavenumber (cm-1)

FIG. 2

Hydrogen-Form
SCM-36
Absorbance (a.u.)
1400
1450
1500
1550
1600
Wavenumber (cm-1)

FIG. 3

SCM-36-SO3H
Intensity (a.u.)
10
20
30
40
50
60
70
2θ (°)

FIG. 4

3.0kV x50.0k SE(U)
1.00um

FIG. 5

SCM-36-SO3H
Absorbance (a.u.)
1400
1450
1500
1550
1600
Wavenumber (cm-1)

FIG. 6

**INTERNATIONAL SEARCH REPORT**

International application No. **PCT/CN2024/122709**

**A. CLASSIFICATION OF SUBJECT MATTER**

B01J29/70(2006.01)i; B01J29/06(2006.01)i; C01B39/04(2006.01)i; C07D307/50(2006.01)i

According to International Patent Classification (IPC) or to both national classification and IPC

**B. FIELDS SEARCHED**

Minimum documentation searched (classification system followed by classification symbols)

IPC: B01J29 C01B39 C07D307

Documentation searched other than minimum documentation to the extent that such documents are included in the fields searched

Electronic data base consulted during the international search (name of data base and, where practicable, search terms used)

CNTXT, ENTXT, ENTXTC, WPABS, WPABSC, WEB OF SCIENCE, CNKI, 万方, WANFANG: 5-羟甲基-2-呋喃甲醛, 沸石, 分子筛, 磺化, 硫化, 硫酸, 糖, 己糖, 果糖, 葡萄糖, 巯基, 巯基乙醇, 巯基乙酸, 羟基甲基糠醛, 羟甲基糠醛, 羟甲基呋喃甲醛, 5-烷氧基甲基-糠醛, 脱水, 氧化, 过氧化氢, 双氧水, 5-Hydroxymethyl-2-furanaldehyde, zeolite, molecularsieve, sulfonated, sulfurized, sulfuricacid, sugar, hexose, fructose, glucose, mercapto, mercaptoethanol, thioglycolicacid, hydroxymethylfurfural, dehydrated, oxidized, hydroxymethylfuranaldehyde, 5-alkoxymethyl-furfural, hydrogenperoxide, HMF, RMF, so3h, H2O2, SCM-36

**C. DOCUMENTS CONSIDERED TO BE RELEVANT**

| Category* | Citation of document, with indication, where appropriate, of the relevant passages | Relevant to claim No. |
|---|---|---|
| A | WANG, Lei et al. "High Selective Production of 5-Hydroxymethylfurfural from Fructose by Sulfonic Acid Functionalized SBA-15 Catalyst" *Composites Part B,* No. 156, 13 August 2018 (2018-08-13), 88-94<br>abstract, and section 2.2 | 1-15 |
| A | CN 116003200 A (CHINA PETROLEUM & CHEMICAL CORP. et al.) 25 April 2023 (2023-04-25)<br>claims 1-10 | 1-15 |
| A | CN 101827833 A (FURANIX TECHNOLOGIES B.V.) 08 September 2010 (2010-09-08)<br>entire document | 1-15 |
| A | CN 104628683 A (DALIAN UNIVERSITY) 20 May 2015 (2015-05-20)<br>entire document | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 November 2024** | **14 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/122709

C. DOCUMENTS CONSIDERED TO BE RELEVANT

| Category* | Citation of document, with indication, where appropriate, of the relevant passages | Relevant to claim No. |
|---|---|---|
| A | CN 107199028 A (CHINA PETROLEUM & CHEMICAL CORP. et al.) 26 September 2017 (2017-09-26) entire document | 1-15 |
| A | CN 115806535 A (DALIAN INSTITUTE OF CHEMICAL PHYSICS, CHINESE ACADEMY OF SCIENCES) 17 March 2023 (2023-03-17) entire document | 1-15 |
| A | CN 116174033 A (NORTHEAST FORESTRY UNIVERSITY) 30 May 2023 (2023-05-30) entire document | 1-15 |
| A | US 2022315550 A1 (ALLIANCE FOR SUSTAINABLE ENERGY, LLC) 06 October 2022 (2022-10-06) entire document | 1-15 |
| A | XING, Xinyi et al. "Sulfonic Acid Functionalized β Zeolite as Efficient Bifunctional Solid Acid Catalysts for the Synthesis of 5-Hydroxymethylfurfural from Cellulose" *International Journal of Biological Macromolecules,* No. 242, 27 May 2023 (2023-05-27), entire document | 1-15 |

Form PCT/ISA/210 (second sheet) (July 2022)

**INTERNATIONAL SEARCH REPORT**
**Information on patent family members**

International application No.
**PCT/CN2024/122709**

| Patent document cited in search report | Publication date (day/month/year) | Patent family member(s) | Publication date (day/month/year) |
|---|---|---|---|
| CN 116003200 A | 25 April 2023 | CN 115959681 A | 14 April 2023 |
| | | TW 202315841 A | 16 April 2023 |
| | | WO 2023061256 A1 | 20 April 2023 |
| | | IN 202447036052 A | 17 May 2024 |
| | | KR 20240076832 A | 30 May 2024 |
| | | SG 11202402424 A | 31 May 2024 |
| | | EP 4417577 A1 | 21 August 2024 |
| | | JP 2024535577 W | 30 September 2024 |
| CN 101827833 A | 08 September 2010 | EA 201070345 A1 | 30 August 2010 |
| | | EA 018486 B1 | 30 August 2013 |
| | | ZA 201001902 B | 29 December 2010 |
| | | CA 2697802 A1 | 12 March 2009 |
| | | CA 2697802 C | 03 April 2018 |
| | | AP 201005179 A0 | 30 April 2010 |
| | | KR 20100072013 A | 29 June 2010 |
| | | EP 2195306 A1 | 16 June 2010 |
| | | EP 2195306 B1 | 17 May 2017 |
| | | AP 201005179 D0 | 30 April 2010 |
| | | MX 2010002603 A | 01 April 2010 |
| | | MY 148864 A | 14 June 2013 |
| | | AU 2008295006 A1 | 12 March 2009 |
| | | AU 2008295006 B2 | 08 November 2012 |
| | | US 2010083565 A1 | 08 April 2010 |
| | | US 8277521 B2 | 02 October 2012 |
| | | JP 2010538033 A | 09 December 2010 |
| | | JP 5950321 B2 | 13 July 2016 |
| | | EA 201101606 A1 | 30 August 2012 |
| | | UA 98002 C2 | 10 April 2012 |
| | | BRPI 0815436 A2 | 23 May 2017 |
| | | BRPI 0815436 B1 | 05 February 2019 |
| | | WO 2009030511 A1 | 12 March 2009 |
| | | SG 159328 A1 | 30 March 2010 |
| | | ID 502547 A | 15 July 2010 |
| | | IN 201001048 P2 | 06 August 2010 |
| | | VN 26344 A | 27 June 2011 |
| | | SG 159328 B | 15 October 2012 |
| | | MX 306818 B | 17 January 2013 |
| | | CN 101827833 B | 10 July 2013 |
| | | VN 10013730 B | 25 March 2015 |
| | | IN 275341 B | 02 September 2016 |
| CN 104628683 A | 20 May 2015 | CN 104628683 B | 08 March 2017 |
| CN 107199028 A | 26 September 2017 | CN 107199028 B | 24 March 2020 |
| CN 115806535 A | 17 March 2023 | CN 115806535 B | 24 September 2024 |
| CN 116174033 A | 30 May 2023 | None | |
| US 2022315550 A1 | 06 October 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4439409 A
- US 6162416 A
- US 4954325 A
- US 5362697 A
- CN 103028424 A
- CN 101812039 A
- CN 115959681 A


### Non-patent literature cited in the description


- *Chinese Journal of Catalysis*, 2020, vol. 41, 1062-1066